(19) Europäisches Patentamt
European Patent Office
Office européen des brevets

(11) Publication number: **0 219 257 B1**

(12) **EUROPEAN PATENT SPECIFICATION**

(45) Date of publication of patent specification: **09.03.94**

(51) Int. Cl.5: **C07D 457/04**, A61K 31/48

(21) Application number: **86307443.1**

(22) Date of filing: **29.09.86**

The file contains technical information submitted after the application was filed and not included in this specification

(54) **Esters of dihydrolysergic acid.**

(30) Priority: **01.10.85 US 782337**
**01.10.85 US 782341**
**01.10.85 US 782342**
**01.10.85 US 782340**
**01.10.85 US 782338**

(43) Date of publication of application:
**22.04.87 Bulletin 87/17**

(45) Publication of the grant of the patent:
**09.03.94 Bulletin 94/10**

(84) Designated Contracting States:
**AT BE CH DE FR GB IT LI LU NL SE**

(56) References cited:
**EP-A- 0 122 044**
**EP-A- 0 218 433**
**CH-A- 0 549 571**
**US-A- 3 580 916**

**CHEMICAL ABSTRACTS, vol. 92, no. 5, 4th February 1980, page 816, abstract no. 42265v, Columbus, Ohio, US; S. JOHNE et al.: "The synthesis of sugar esters of lysergic and 9,10-dihydrolysergic acid"**

(73) Proprietor: **ELI LILLY AND COMPANY**
**Lilly Corporate Center**
**Indianapolis Indiana 46285(US)**

(72) Inventor: **Garbrecht, William Lee**
**7303 Woodstream Drive**
**Indianapolis Indiana 46254(US)**
Inventor: **Marzoni, Gifford Purnell**
**2612 Amherst Street**
**Indianapolis Indiana 46268(US)**
Inventor: **Parli, Carol John**
**3860 Glencairn Lane**
**Indianapolis Indiana 46226(US)**
Inventor: **Whitten, Kathleen Rose**
**9302 East 180 South**
**Zionsville Indiana 46077(US)**
Inventor: **Cohen, Marlene Lois**
**521 Oakwood Drive**
**Indianapolis Indiana 46260(US)**
Inventor: **Fuller, Ray Ward**
**7844 Singleton Drive**
**Indianapolis Indiana 46227(US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

CHEMICAL ABSTRACTS, vol. 89, no. 24, 11th December 1978, page 620, abstract no. 215637m, Columbus, Ohio, US; R. RUCMAN et al.: "Derivatives of lysergic acid. Part V. Synthesis of the 1,8-dialkyl derivative of dihydrolysergic acid"

JOURNAL OF MEDICINAL CHEMISTRY, vol. 31. no. 2, February 1988, pages 444-448, American Chemical Society, US; W.L. GARBRECHT et al.: "(8beta)-Ergoline-8-carboxylic acid cycloalkyl esters as serotonin antagonists: Structure-activity study"

JOURNAL OF MEDICINAL CHEMISTRY, vol. 31, no. 2, February 1988, pages 444-448

## Description

United States Patent No. 3,580,916 discloses a group of lysergic (I) and 9,10-dihydrolysergic acid (II) esters formed with various open chain and cyclic diols having the following structures:

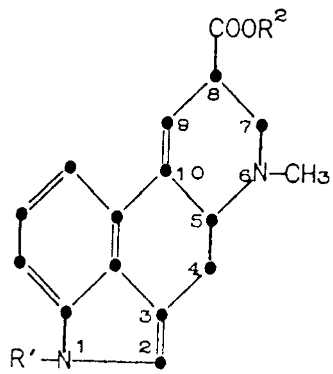

and

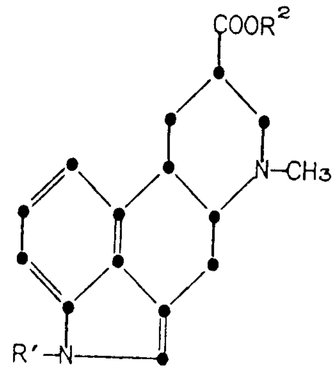

(I) (II)

wherein R' is H, $C_1$-$C_3$ alkyl, allyl or benzyl and $R^2$ is $C_2$-$C_8$ monohydroxyalkyl, $C_2$-$C_8$ dihydroxyalkyl or $C_5$-$C_{11}$ monohydroxycycloalkyl having from 5-8 ring carbons. The compounds were found to be useful as serotonin antagonists. European Patent Publication 0 122 044 discloses the use of such compounds as $5HT_2$ receptor blockers.

The present invention provides ergolines of Formula (III):

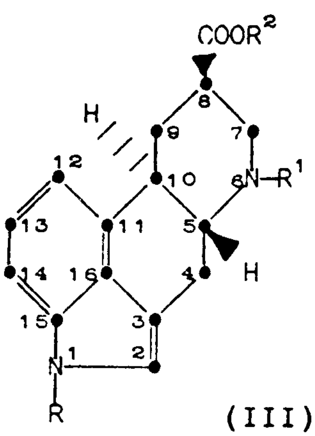

(III)

wherein R is primary or secondary $C_1$-$C_8$ alkyl, $C_2$-$C_4$ alkenyl-$CH_2$, $C_3$-$C_8$ cycloalkyl or $C_3$-$C_6$ cycloalkyl-substituted $C_1$-$C_5$ primary or secondary alkyl, the total number of carbon atoms in R not to exceed 8; $R^1$ is allyl, H or $C_1$-$C_4$ straight-chain alkyl; ie., methyl, ethyl, n-propyl or n-butyl, and $R^2$ is $C_1$-$C_3$ alkoxy-$C_5$-$C_7$ cycloalkyl; $C_5$-$C_7$ cycloalkyl or keto-substituted $C_5$-$C_7$ cycloalkyl; or $C_3$-$C_7$ ketoalkyl

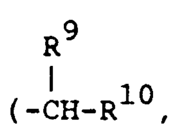

wherein $R^9$ is H, methyl, or ethyl and $R^{10}$ is a $C_2$-$C_5$ alkyl group containing a ketone moiety) attached to the acidic function through a primary or secondary carbon; and pharmaceutically-acceptable acid addition salts thereof.

Compounds of Formula (III), wherein $R^1$ is other than H, are central or peripheral serotonin $5HT_2$ receptor antagonists. Compounds wherein $R^1$ is H are primarily useful as intermediates.

Groups which R represents include methyl, ethyl, allyl, n-propyl, isopropyl, crotyl, methallyl, n-hexyl, sec-amyl, sec-octyl, n-heptyl, 2,4-dimethyl-pentyl, 2-ethylpentyl, cyclopropyl, cyclopropylmethyl, cyclopentyl methyl, 2-cyclobutyl ethyl, cyclohexyl, isobutyl, sec.-butyl, 3-methyl-2-butyl, isoamyl, 2-methylhexyl, 3-methylhexyl, 4-methylhexyl(isohexyl), 2-hexyl, 3-hexyl, n-heptyl, 2-heptyl, 3-heptyl, 4-heptyl, n-octyl, 2-octyl, 3-octyl, 4-octyl, isooctyl, 2-methylheptyl, 3-methyl-2-heptyl, and the like. Illustrative of the groups which $R^2$ represents include 4-methoxycyclohexyl, 3-ethoxycyclohexyl, 3-methoxycyclopentyl, 3-methoxycycloheptyl, 3-n-propoxycycloheptyl, 3-ethoxycyclopentyl, 4-isopropoxycyclohexyl, 2-methoxycycloheptyl 2-oxopropyl, 1-methyl-2-oxopropyl, 1-ethyl-2-oxopropyl, 1-methyl-2-oxobutyl, 1-ethyl-2-oxobutyl, 1-methyl-3-oxobutyl, 1-ethyl-3-oxobutyl, cyclohexyl, 3-ketocyclohexyl, cyclopentyl, 3-ketocycloheptyl, cycloheptyl, 3-ketocyclopentyl, 4-ketocyclohexyl, 2-ketocycloheptyl,

Compounds according to the above formula are named as ergoline derivatives in which the trans(-) or 5R,10R configuration of the bridgehead hydrogens is specified (The same configuration as in the naturally-occurring 9,10-dihydro ergot alkaloids). In United States Patent 3,580,916, a different naming system is used; the basic ring system is named as a 6aR,10aR-4,6,6a,7,8,9,10,10a-octahydroindolo[4,3-f,g]-quinoline. Illustratively, by the alternate naming system 9,10-dihydrolysergic acid becomes 6aR,10aR-7-methyl-4,6,6a,7,8,9,10,10a-octahydroindolo[4,3-f,g]quinoline-9β-carboxylic acid. Another equally valid name for dihydrolysergic acid is 6-methyl-8β-carboxyergoline. The trivial name "ergoline" is used herein with the numbering system specified in (III) above for compounds in which $R^1$ is other than methyl and the 9,10-dihydrolysergic acid nomenclature for 6-methyl derivatives.

In addition, in 9,10-dihydrolysergic acid, the C-8 carboxyl is beta or R. Thus, again using the ergoline naming system, derivatives of 9,10-dihydrolysergic acid become derivatives of 5R,8R,10R(or 5β,8β,10α) 6-methylergoline-8β-carboxylic acid.

While the configuration at asymmetric carbons 5,8 and 10 in Formula (III) is set (5β,8β and 10α), generally speaking, the alkoxycycloalkyl ester group (or hydroxycycloalkyl group) contains two additional asymmetric carbons. For example, 3-methoxycyclohexanol exists as two racemates, each racemate containing two enantiomers or stereoisomers. However, where the alkoxycycloalkanol (or hydroxycycloalkyl group) possesses a plane of symmetry, as in a 4-alkoxycyclohexanol, mirror images turn out to be superimposable, and the compound actually exists in only two forms. These forms are designated as the cis form and the trans form, drawn for convenience in two dimensions as (IVa) and (IVb).

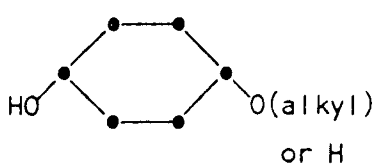

cis

(IVa)

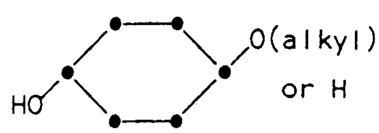

trans

(IVb)

When a monoester of a 1-substituted-9,10-dihydrolysergic acid is formed with a cis or trans 4-alkoxycycloalkanol, the product will be a single geometrical isomer. In general, the two esters in this instance will also be named, for the sake of simplicity, as cis and trans 4-alkoxycyclohexyl (or 4-hydroxycycloalkyl) esters.

This invention contemplates all such forms useful as peripheral serotonin antagonists; that is, the individual diastereoisomers and geometrical isomers as well as racemates.

Preferred compounds of the invention include those having one or more of the following features:

(A) $R^2$ is $C_1$-$C_3$ alkoxy-$C_5$-$C_7$ cycloalkyl;

(B) $R^2$ is $C_5$-$C_7$ cycloalkyl or keto-substituted $C_5$-$C_7$ cycloalkyl;

(C) $R^2$ is $C_3$-$C_7$ ketoalkyl;

(D) $R^1$ is methyl;

(E) $R^2$ is trans 4-methoxycyclohexyl;

(G) R is isopropyl;

Pharmaceutically-acceptable acid addition salts of the compounds of Formula (III) include salts derived

from non-toxic inorganic acids such as: hydrochloric acid, nitric acid, phosphoric acid, sulfuric acid, hydrobromic acid, hydriodic acid, phosphorous acid and the like, as well as salts derived from non-toxic organic acids such as aliphatic mono and dicarboxylic acids, phenyl-substituted alkanoic acids alkanoic and alkandioic acids, aromatic acids, aliphatic and aromatic sulfonic acids, etc. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methoxybenzoate, phthalate, terephthalate, benzenesulfonate, toluenesulfonate, chlorobenzenesulfonate, xylenesulfonate, phenylacetate, phenylpropionate, phenyl-butyrate, citrate, lactate, $\beta$-hydroxybutyrate, glycollate, malate, tartrate, methanesulfonate, propanesulfonate, naphthalene-1-sulfonate, naphthalene-2-sulfonate and the like salts.

Illustrative compounds of this invention include:

1-methyl-6-ethyl-8$\beta$-(2-methoxy)cyclopentyloxycarbonylergoline hydrochloride
1-n-propyl-6-allyl-8$\beta$-(3-ethoxy)cycloheptyloxycarbonylergoline sulfate
4-methoxycyclohexyl 1-methyl-9,10-dihydrolysergate phosphate
3-methoxycyclohexyl 1-n-octyl-9,10-dihydrolysergate maleate
1-isopropyl-6-n-propyl-8$\beta$-(2-n-propoxy)cyclohexyloxycarbonylergoline hydrobromide
1-allyl-6-ethyl-8$\beta$-(4-ethoxy)cycloheptyloxycarbonylergoline succinate
1,6-diethyl-8$\beta$-(2-keto)propyloxycarbonylergoline succinate
1-methyl-6-ethyl-8$\beta$-(1-methyl-2-keto)butyloxycarbonylergoline hydrochloride
1,6-diethyl-8$\beta$-cyclohexyloxycarbonylergoline succinate
1-methyl-6-ethyl-8$\beta$-cyclopentyloxycarbonylergoline hydrochloride
1-n-propyl-6-allyl-8$\beta$-cycloheptyloxycarbonylergoline sulfate
1-isopropyl-6-n-propyl-8$\beta$-(2-oxo)cyclohexyloxycarbonylergoline hydrobromide
1-allyl-6-ethyl-8$\beta$-(4-oxo)cycloheptyloxyergoline tartrate
1-n-propyl-6-allyl-8$\beta$-(1-ethyl-3-keto)butyloxycarbonylergoline sulfate
1-isopropyl-6-n-propyl-8$\beta$-(1-methyl-2-keto)-propyloxycarbonylergoline hydrobromide
3-oxobutyl 1-methyl-9,10-dihydrolysergate
4-oxopentyl 1-ethyl-9,10-dihydrolysergate
1-methyl-4-oxopentyl 1-allyl-9,10-dihydrolysergate
1-allyl-6-ethyl-8$\beta$-(3-keto)butyloxyergoline tartrate and the like.

The preparation of compounds represented by Formula (III) above can be accomplished by the general method of United States Patent 3,580,916. According to this procedure, dihydrolysergic acid is first alkylated on the indole nitrogen using standard procedures, e.g., base plus an alkyl halide. Liquid ammonia is a convenient solvent with sodamide as the base and primary or secondary $C_1$-$C_8$ alkyl, $C_3$-$C_8$ cycloalkyl or $C_3$-$C_6$ substituted $C_1$-$C_5$ primary or secondary alkyl iodide or a $C_2$-$C_4$ alkenyl chloride or bromide as the alkylating agent. (See also United States Patent 3,183,234, which contains general directions and a specific example of the above alkylation procedure).

An alternate indole-N-alkylation procedure whereby an arylsulfonate is used in the presence of an alkali metal hydroxide is more fully described in United States Patent 4,734,501. According to this procedure, for example, an arylsulfonate of the structure R-O-$SO_2$-phenyl-Y, wherein arylsulfonate of the structure R-O-$SO_2$-phenyl-Y, wherein Y is H, 4-$CH_3$, 4-Br, or 4-$NO_2$, can be reacted with 9,10-dihydrolysergic acid in an aprotic solvent, conveniently DMSO, in the presence of sodium or potassium hydroxide, to yield the desired 1-N-alkylated product.

With the indole nitrogen substituent in place, the next step in the synthetic procedure is esterification. This procedure requires relatively mild reaction conditions as noted in United States patent 3,580,916. The reaction is an otherwise standard acid-catalyzed esterification. The free acid prepared above and compounds of formula $R^2OH$ are the reactants and a convenient work-up of the esterification mixture involves partitioning between water and a water-immiscible solvent; [(ethylene dichloride)/$H_2O$], for example.

A preferred procedure, utilizing a further aspect of this invention, is to use a novel synthetic step whereby the free 9,10-dihydrolysergic acid is reacted with a sulfonate of the formula $R^2$-O-$SO_2$-Z, wherein Z is $C_1$-$C_3$ alkyl, phenyl or substituted phenyl wherein said substituents can be lower alkyl ($CH_3$, $C_2H_5$), nitro, halo(Br,Cl), alkoxy ($CH_3O$) and the like, in the presence of excess potassium carbonate or similar base in an aprotic solvent. The tosylate ester is preferred. The quantity of base must be sufficient to form a salt with the lysergic acid carboxyl group plus sufficient excess to scavenge any sulfonic acid by-product. This process is applicable to the preparation of alkoxycycloalkyl esters, cycloalkyl and ketocycloalkyl esters, and in fact is generally applicable to the preparation of carbocyclic acid esters of $C_5$-$C_7$ cycloalkanols.

Procedures hitherto available for the preparation of such esters have given low yields, and in some instances, esterification was not observed--see Shaw et al., J. Org. Chem., 43, 1017 (1978) 39, 1968 (1974), Sowinski et al, ibid, 44, 2369 (1979), Pfeffer et al Tetrahedron Letters, 4063 (1972) or Liotta et al, ibid, 2417 (1974).

An alternative procedure for the preparation of compounds of Formula (III) wherein $R^2$ is $C_3$-$C_6$ ketoalkyl involves the acid-catalyzed esterification of the N-alkylated dihydrolysergic acid with an alcohol of formula $R^5OH$, wherein $R^5$ is a $C_3$-$C_6$ hydroxyalkyl group

$$(-\overset{\overset{R^{9}}{|}}{C}H-R^{12},$$

wherein $R^9$ is as defined above and $R^{12}$ is a $C_2$-$C_5$ alkyl group containing a secondary hydroxy group). As noted above, a convenient workup of the esterification product involves partitioning between water and a water-immiscible solvent.

Next, the secondary alcohol group of the ester can be oxidized to the ketone, yielding the ketoalkyl esters of this invention where $R^1$ is methyl (esters of 9,10-dihydrolysergic acid). It should be noted that, if the alkanediol ($R^5OH$) is not symmetrical, the resulting ester may be a mixture; ie., if the diol is 1-ethyl-2-hydroxypropanol, a mixture of the 1-ethyl-2-hydroxypropyl and 1-methyl-2-hydroxybutyl esters will result. These esters can be separated mechanically, or the mixture can be oxidized and the keto esters separated. However, since differences in pharmacologic activity between any two such isomeric esters would not be expected to be large (less than an order of magnitude), the mixture could be employed as such. However, it will be apparent that use of a symmetrical diol such as butane-2,3-diol would be preferred since its use would avoid the aforesaid isomer problem. However, if the diol contains a primary alcohol, reaction conditions can be employed which favor reaction on the primary alcohol group; for example secondary alcohols typically react more slowly than primary alcohols in a standard acid-catalyzed esterification.

Suitable oxidizing agents for the final step include acetic anhydride/DMSO, dicyclohexylcarbodiimide, chromate salts, positive halogen agents such as $Ca(OCl)_2$, NaOCl and the like.

If the desired final product is not a 9,10-dihydrolysergic acid ester (ie; not a 1-R-6-methylergoline-8β-carboxylic acid ester), but is a 6-ethyl, 6-n-propyl, 6-n-butyl, 6-allyl or the like derivative, the replacement of the 6-methyl group may take place prior to the final esterification. In this procedure, a lower alkyl (methyl or ethyl) ester of a 1-R-9,10-dihydrolysergic acid can be conveniently utilized. Replacement of the 6-methyl group with ethyl, n-propyl, allyl, n-amyl, n-butyl, n-hexyl, or the like, can then be carried out by the procedure of Kornfeld and Bach, United States Patent 4,166,182, whereby the N-methyl group is reacted with cyanogen bromide to form an N-cyano derivative. The cyano group can be removed by hydrogenation using zinc dust and hydrochloric acid. Alternatively, basic hydrolysis can be used. Either procedure provides a secondary amine group at 6, but also a free 8β-carboxylic acid, because the hydrolysis also saponifies the 8β-lower alkyl ester group. Next, re-esterification with the desired $R^2OH$ alkanol can be carried out followed by alkylation or allylation at N-6 using an allyl chloride or alkyl iodide in the presence of base, conveniently in DMF (dimethylformamide).

This procedure is graphically illustrated in Reaction Scheme 1 below.

**Reaction Scheme 1**

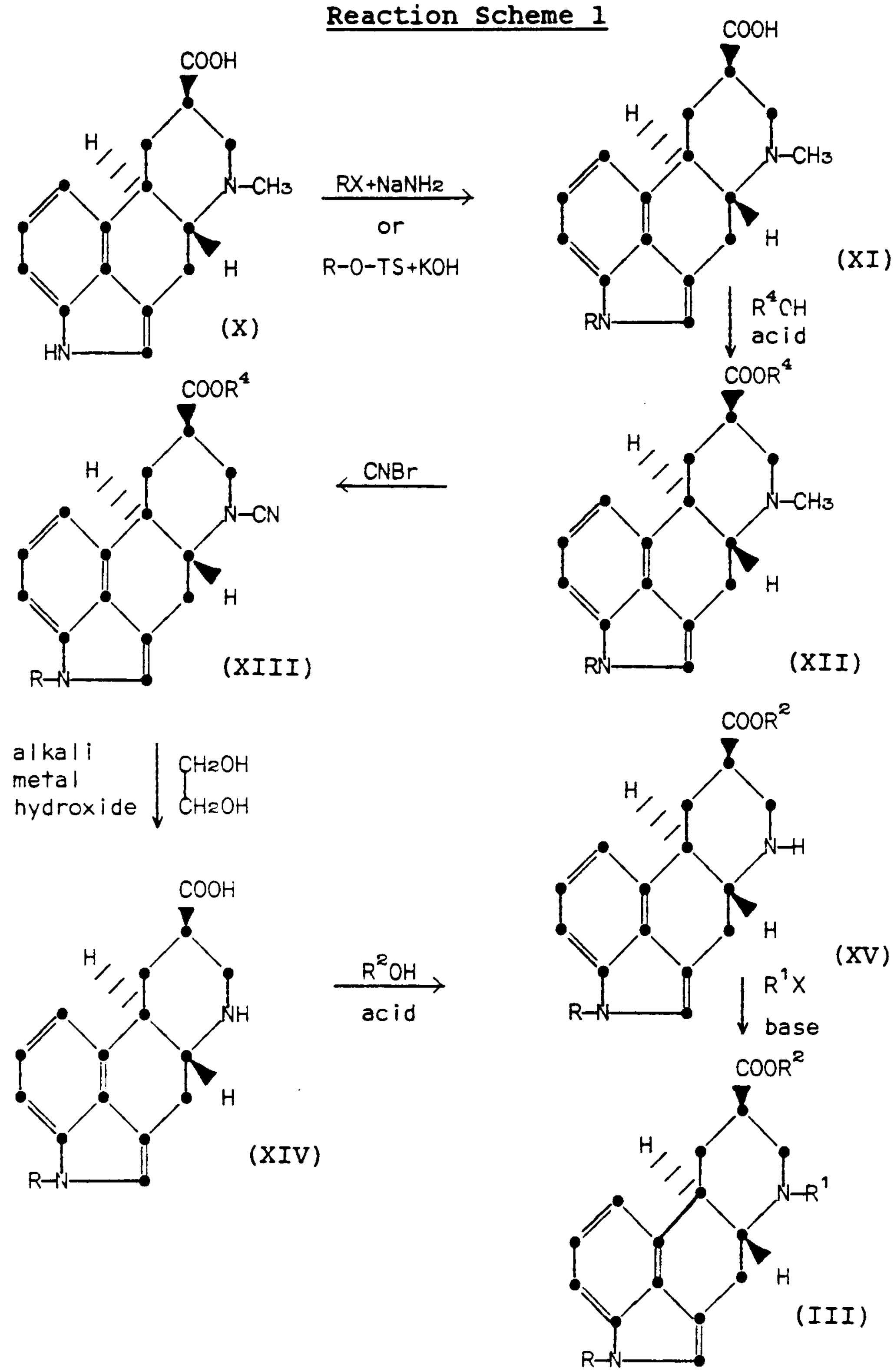


(X) (XI) (XII) (XIII) (XIV) (XV) (III)

More specifically, in the above Reaction Scheme 1, 9,10-dihydrolysergic acid (X) is alkylated on the indole nitrogen with an alkyl ($C_1$-$C_8$ alkyl) halide, a $C_2$-$C_4$ alkenyl-$CH_2$ halide, a $C_3$-$C_6$ cycloalkyl halide or a $C_3$-$C_6$ cycloalkyl-$C_1$-$C_5$ alkyl halide, using a strong base such as sodamide to create the reactive anion or

preferably using an aryl sulfonate such as a p-tosylate in the presence of potassium hydroxide in DMSO. The $N^1$ product (XI) is then esterified with a lower alkanol $R^4OH$ (a $C_1$-$C_2$ alkanol preferably) to yield the 1-R ester (XII). This intermediate is then reacted with CNBr by standard procedures to replace the methyl group and form an 6-cyano derivative (XIII). Removal of the cyano group under the preferred basic conditions yields a 1-substituted-9,10-dihydro-6-desmethyllysergic acid (XIV), because the basic conditions also saponifies the C-8 ester group. Next, the 1-R-6-desmethyldihydrolysergic acid is re-esterified with a compound of formula $R^2OH$ or a tosyl ester thereof to yield the $N^6$-desmethyl ester (XV). The piperidine ring nitrogen ($N^6$) is then realkylated with a $C_1$-$C_4$ alkyl or allyl halide in the presence of base under standard conditions to yield the compounds of this invention (III).

It might seem redundant to realkylate at $N^6$ with a methyl group since that group is present in the 9,10-dihydrolysergic acid starting material. However, this process would enable one to insert a radiolabeled ($C^{14}$ or $H^3$) methyl group suitable for metabolic studies.

The alternative procedure for preparation of the compounds of Formula (III) wherein $R^2$ is $C_3$-$C_6$ ketoalkyl, is more specifically outlined below in Reaction Scheme 2:

**Reaction Scheme 2**

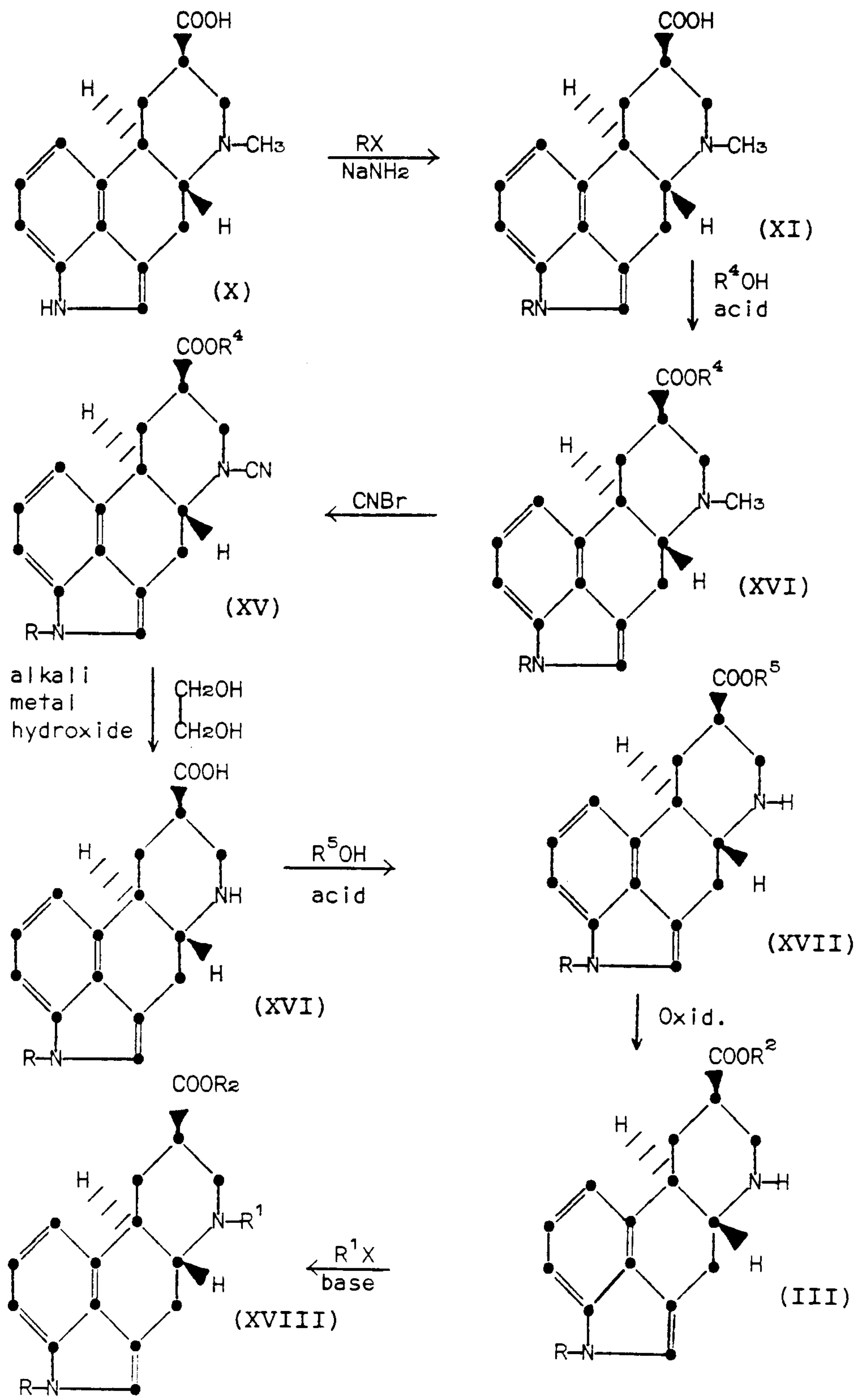


wherein $R^4$ is $C_1$-$C_2$ alkyl, $R^1$ and R are as previously defined and $R^5$ is hydroxy $C_3$-$C_6$ alkyl in which the hydroxyl group is a secondary hydroxyl group.

In Reaction Scheme 2, 9,10-dihydrolysergic acid (X) is alkylated on the indole nitrogen with an R-hal where "hal" is a halide such as I, Cl, or Br, using a strong base such as sodamide to create the reactive anion. Alternatively, an aryl sulfonate, R-O-$SO_2$-phenyl-Y, can be reacted with the indole nitrogen in the

presence of an alkali metal halide in an aprotic solvent. The N-1 alkyl or allyl product (XI) is then esterified with a lower alkanol $R^4OH$ (a $C_1$-$C_2$ alkanol preferably) to yield the N-1 alkylated ester (XIV). This compound is then reacted with CNBr by standard procedures to replace the N-6 methyl group and form an N-cyano derivative (XV). Removal of the cyano group under the preferred basic conditions yields a 1-R-9,10-dihydro-6-desmethyllysergic acid (XVI), because the basic conditions also saponifies the C-8 ester group. Next, the 1-R-6-desmethyldihydrolysergic acid (or 1-R-8$\beta$-carboxyergoline) is re-esterified with a desired alkanediol ($R^5OH$) to yield the N-6-desmethyl ester (XVII). The secondary hydroxyl in the side chain is now oxidized to a ketone group to yield an ergoline ketoalkyl ester (XVIII). The piperidine ring nitrogen (N-6) is then realkylated with a $C_1$-$C_4$ alkyl or allyl halide and base under standard conditions to yield the compounds of Formula (III) wherein $R^2$ is $C_3$-$C_6$ ketoalkyl.

The above procedure is particularly useful where the esterifying alcohol $R^5OH$ is symmetrical. Alternate procedures are available for the preparation of ketoalkyl esters of 1-alkyl or allyl-9,10-dihydrolysergic acid and related N-6 homologues whereby a keto alcohol is used directly to yield a compound according to III. Alternatively, a "protected" $C_{3-6}$ keto alcohol can be used to form the ester; ie., a ketal of 2-keto propanol can be formed with ethylene glycol. The primary hydroxyl can then be replaced by chloro (using $SOCl_2$) and the ketal chloride reacted with the sodium salt of the 1-R-9,10-dihydrolysergic acid. The ketal protecting group is removed by treatment with acid. Obviously, the usual acid catalyzed esterification could not be used with an ketalalkanol because the ketal group would come off during such esterification and alternate procedures available in the art should be used. Also, if it is desirable to avoid an acid catalyzed reaction with a ketoalkanol, a carboxy activating group can be employed to form the ester under non-acidic conditions; ie., acid chloride or bromide with base. Also carboxy activating agents such as carbodiimide and azolide N,N'-carbonylimidazole can be employed.

Alternatively, a keto alcohol in which the ketone group is protected, as by ketal formation, can be employed in the esterification procedure in Reaction Scheme 2, to yield an ergoline ester lacking an N-6 substituent, analogous to Formula (XVII). Alkylation or allylation at N-6 then yields a further intermediate containing an ester with a protected keto group, (XIX)

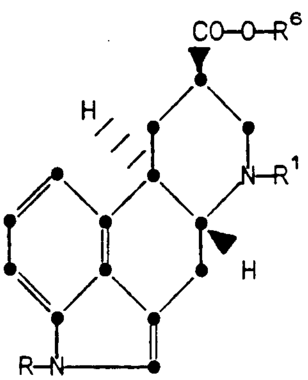

**(XIX)**

wherein $R^6$ is a $C_3$-$C_6$ protected keto alkyl

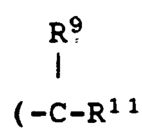

wherein $R^9$ has its previous meaning and $R^{11}$ is $C_2$-$C_5$ alkyl containing a protected keto function; for example

$-CH_2-C(OC_{1-2}\ alk)_2-CH_3$ or 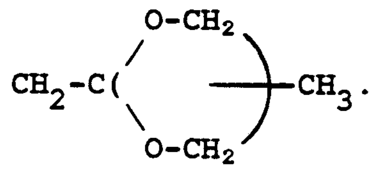

Treatment of (XIX) with acid then removes the protecting group to yield a ketoalkyl ester of a 1-substituted-9,10-dihydrolysergic acid or a 1-substituted-6-alkyl (or allyl) ergoline-8β-carboxylic acid.

The $C_3$-$C_6$ alkanediols ($R^5OH$) used to esterify the 1-substituted-9,10-dihydrolysergic acid (or N-6 homologues or congeners thereof) as the last intermediate prior to oxidation of the secondary alcohol to a ketone, will have at least one center of asymmetry, the carbon carrying the secondary alcohol. The diol will also have a second asymetric carbon if the other hydroxyl is secondary. Where the esterifying hydroxyl is primary, the final keto alkyl ester will not have an asymetric center since the asymmetric center in the starting alcohol will be removed by the oxidative process. If the esterifying hydroxyl is secondary, however, the final product will have four asymetric carbons C-6, C-8, C-10 and the side chain asymmetric carbon attached to the carboxyl oxygen. The 9,10-dihydrolysergic acid or ergoline-8β-carboxylic acid asymetric carbons are all R and the side chain carbon in the ketoalkyl group can be S or R. The parent alcohol will then have four stereoisomers, RR, RS, SR and SS. Where the diol is symmetrical, as in butane-2,3-diol, a plane of symmetry exists and there are only three stereoisomers, RR, SS and RS (same as SR). However, esterification removes the plane of symmetry and there are two more diastereoisomers (as with a non-symmetrical diol) designated as RRR-RR, RRR-SR, RRR-RS, and RRR-SS yielding only two keto esters after oxidation (RRR-S and RRR-R).

Finally, one skilled in the art will appreciate that the above processes can also be carried out on the corresponding 9-10 unsaturated lysergic acid derivatives. In this regard, a final step of standard Pd/C catalyzed hydrogenation would yield the compounds of the present invention.

According to one aspect of the invention there is provided a process of preparing a compound of Formula (III) which comprises:

A) esterifying the 8-carboxylic acid function of a compound of Formula (V):

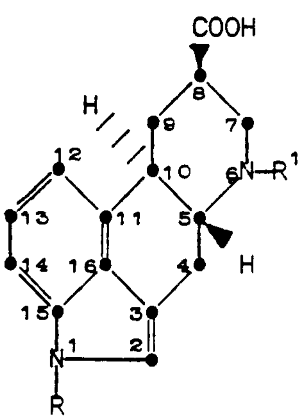

(V)

wherein R and $R^1$, are as defined in above; or

B) oxidizing a compound of Formula (VI):

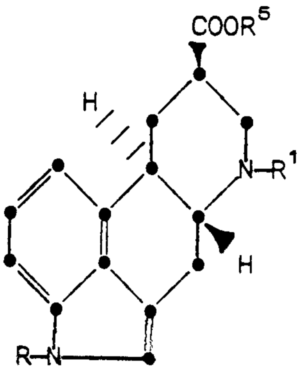

(VI)

wherein $R^5$ is as defined in above

C) alkylating a compound of Formula (III):

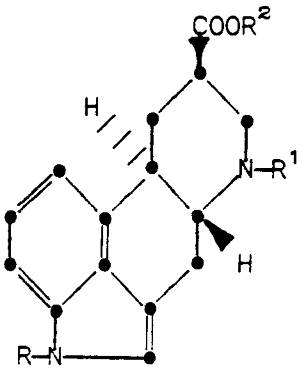

(III)

wherein one only of R and $R^1$ is hydrogen and $R^2$ is as defined above; or,

D) hydrogenating a compound of Formula (VIII):

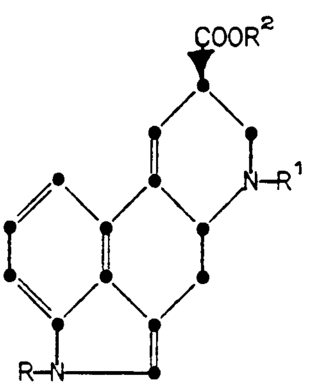

(VIII)

The following examples illustrate the process and compounds of the present invention.

Example 1

Preparation of Trans-4-methoxycyclohexyl 1-Isopropyl-9,10-dihydrolysergate.

A reaction mixture, prepared from 1 g of 1-isopropyl-9,10-dihydrolysergic acid, 1.77 g of potassium carbonate and 15 ml of DMF, was heated to about 70 °C. 3.28 g of cis-4-methoxycyclohexyl tosylate were

added. After about 18 hours, at 70°C, HPLC (reverse phase, 3:1 acetonitrile/0.1M aqueous ammonium acetate) indicated that the reaction was about 87% complete. The reaction mixture was then partitioned between 100 ml of distilled water and 100 ml of ethyl acetate. TLC (Chloroform/methanol/acetic acid, 18:6:1) indicated no desired product in the aqueous layer. The organic layer was extracted twice with 50 ml portions of distilled water and was then dried. Evaporation of the solvent gave 1.37 g of a 16:84 mixture of the cis and trans-4-methoxycyclohexyl 1-isopropyl-9,10-dihydrolysergate. The residue was dissolved in 15 ml of anhydrous ethanol containing .37 g of maleic acid. 250 ml of diethyl ether were added, whereupon a crystalline maleate began to form. The mixture was chilled overnight at about 0°C and was then filtered. The filter cake was washed with ether and then dried. Assay indicated 91.4% trans and 8.6% cis esters; wt = .86 g. This residue was dissolved in 35 ml of anhydrous ethanol and 400 ml of ether added. This time, the crystalline product was 8.4% cis and 93.6% trans; wt = .74 g. Recrystallization of this residue from ethyl acetate/toluene gave a residue which contained 5.6% cis and 94.4% trans esters. This residue was dissolved in 27 ml of anhydrous ethanol and 300 ml of ether added. Crystals thus produced weighed .48 g and contained 3.6% cis and 96.4% trans ester. The process was repeated using 21 ml of anhydrous ethanol and 250 ml of ether. 0.38 g of 4-methoxycyclohexyl 1-isopropyl-9,10-dihydro-lysergate maleate were obtained which contained 2% cis and 98% trans ester; mp = 172-173°C; molecular ion (free base) at 424.

| | | | | |
|---|---|---|---|---|
| Analysis: | Calc.: | C, 66.65; | H, 7.46; | N, 5.18; |
| | Found: | C, 66.50; | H, 7.56; | N, 5.08. |

Example 2

Preparation of Cis-4-methoxycyclohexyl-1-Isopropyl-9,10-dihydrolysergate.

4-Methoxycyclohexanol (27.9 g) was reacted with 1-isopropyl-9,10-di-hydrolysergic acid (6.24 g) and p-toluenesulfonic acid (3.8 g) at room temperature for 3 days. It was then heated at about 90°C for 3 hours. At this time, HPLC analysis showed 19% unreacted lysergic acid, 70% 4-methoxycyclohexyl ester and 4% desmethyl compound (4-hydroxycyclohexyl ester). The reaction mixture was dissolved in $(CH_2Cl)_2$ and the organic solution washed with dilute ammonium hydroxide (pH ≅ 10). The crude product was isolated by evaporation of the organic solvent. It was treated with an excess of maleic acid and the maleate salt thus formed was crystallized from methanol/ether. The crystals were dissolved in boiling methanol, the hot solution treated with decolorizing charcoal and filtered. Addition of ether to the filtrate yielded 1.2 g of crystalline cis-4-methoxycyclohexyl 1-isopropyl-9,10-dihydrolysergate maleate (90% pure). A second crop, weight 2.26 g, was shown by HPLC to be 86% pure. The combined fractions were purified by preparative HPLC [C-18, 50:50 $CH_3CN/NH_4OAc$ (1)]. The maleate salt was reformed. Recrystallization from methanol/ether gave 1.27 g of 99% pure cis isomer; molecular ion at 424.

| | | | | |
|---|---|---|---|---|
| Analysis: | Calc.: | C, 66.65; | H, 7.46; | N, 5.18; |
| | Found: | C, 66.38; | H, 7.74; | N, 5.37. |

The preparation of starting materials is illustrated below.

Preparation 1

Cis-4-methoxycyclohexyl Tosylate.

A solution was prepared by dissolving 65.45 g of 4-methoxycyclohexanol in 81 ml of pyridine. The solution was cooled to about 10°C. 105.4 g of p-toluene sulfonyl chloride were added in batchwise fashion over 15 minute period. The reaction mixture was stirred for 1 hour in the range 10-20°C. and 4 hours at 25-30°C. at which time it was added to 500 ml of an ice/water mixture containing 100 ml of 12N hydrochloric acid. A precipitate which resulted was separated by filtration, and the filter cake washed with water. The wet filter cake was slurried with 300 ml of anhydrous ethanol. The slurry was warmed on the steam bath and then cooled at about 0°C. The chilled slurry was filtered and the filter cake washed with cold anhydrous ethanol; yield = 106.9 g; 82.4% cis ester by HPLC (C-18; 60:40 methanol/$H_2O$). Recrystallizations from pet.

ether gave 81.62 g of 93.6% cis ester. A second recrystallization from anhydrous ethanol yielded 72.04 g of 98% cis 4-methoxycyclohexyltosylate melting at 85-7 °C.

| | | | | |
|---|---|---|---|---|
| Analysis: | Calc.: | C, 59.13; | H, 7.09; | N, 11.28; |
| | Found: | C, 59.32; | H, 7.20; | N, 11.49. |

Preparation 2

Preparation of Cis-4-methoxycyclohexanol.

Following the procedure of J. Org. Chem., 28 1923 (1963), 13.35 g of $AlCl_3$ and 125 ml of ether were stirred under $N_2$. 25 ml of 1M $LiAlH_4$ in ether were added by syringe to the solution. Next, 13 g of 4-methoxycyclohexanol in 50 ml of ether were added to the stirred mixture over a 30 minute period. The mixture was then allowed to settle. The supernate was removed. The solid remaining was washed three times with 25, 50 and 50 ml portions of ether. The solid precipitate was filtered and the filter cake thoroughly washed with ether. (The filtrate, and washings contained trans-4-methoxycyclohexanol). The dried precipitate (19.17 g) was slurried in 100 ml of ether. 100 ml of 10% sulfuric acid were slowly added thereto (30 minutes). The cis isomer obtained from the decompositions of the $AlCl_2$ complex was in the ether layer which was separated. The separated layer was washed successively with 100 ml water, 50 ml saturated aqueous sodium bicarbonate and 50 ml of brine. The ethereal solution was dried and the ether evaporated to yield 1.7 g of cis-4-methoxycyclohexanol. An additional 4 g were obtained from the water layer.

Example 3

Preparation of R-1-Methyl-2-oxopropyl-1-isopropyl-9,10-dihydrolysergate

Two-thirds of a gram of R,R-1-methyl-2-hydroxypropyl-1-isopropyl-9,10-dihydrolysergate maleate was partitioned between 50 ml of ethylene dichloride and 50 ml of saturated aqueous sodium bicarbonate. The organic layer, shown by TLC (chloroform/methanol/acetic acid, 18:6:1) to be one-spot material, was dried and the solvent evaporated in vacuo. The crystalline residue (.38 g) was mixed with 1 ml DMSO (dimethylsulfoxide) and 7 ml of acetic anhydride. The reaction mixture was stirred overnight at room temperature. HPLC indicated absence of starting material. 10 ml of ethanol were added and the mixture stirred for one additional hour. The oxidation mixture was partitioned between 75 ml of ethylene dichloride and 75 ml of saturated aqueous sodium bicarbonate. The ethylene dichloride layer was separated and dried. The residue was purified using preparative HPLC over C-18 reverse phase silica; (acetonitrile/water/triethylamine 65:35:0.02;) 100 ml/min; 200 ml fractions. Fractions 9-20, containing R-1-methyl-2-oxopropyl 1-isopropyl-9,10-dihydrolysergate formed in the above oxidation, were combined, partially evaporated and extracted twice with 200 ml portions of ethylene dichloride, and dried. Evaporation of the solvent from the combined extracts gave 0.38 g of ester. The ester was converted to the corresponding maleate salt by dissolving 0.38 g of the free base in 10 ml of ethyl acetate. 0.13 g of maleic acid were added to the solution. A crystalline salt started to precipitate. 100 ml of ether were added and the crystallization mixture chilled (about 0 °C) overnight. Crystals were separated by filtration, and the filter cake washed with ether and dried. Recrystallization from ethyl acetate gave 150 mg of R-1-methyl-2-oxopropyl 1-isopropyl-9,10-dihydrolysergate maleate.

| | | | | |
|---|---|---|---|---|
| Analysis: | Calc.: | C, 65.04; | H, 6.87; | N, 5.62 |
| | Found: | C, 64.83; | H, 7.10; | N, 5.43. |

The above procedure was carried out on S,S-1-methyl-2-hydroxypropyl 1-isopropyl-9,10-dihydrolysergate. .54 g of the maleate salt of this alcohol were converted to the free base and the free base oxidized with acetic anhydride-DMSO reagent to yield .36 g of S-1-methyl-2-oxopropyl 1-isopropyl-9,10-dihydrolysergate which free base was converted to the maleate salt by the procedure of the above example; yield of maleate salt = 170 mg; mass spectrum - molecular ion (of free base) at 382.

| | | | | |
|---|---|---|---|---|
| Analysis: | Calc.:<br>Found: | C, 65.04;<br>C, 64.80; | H, 6.87;<br>H, 7.11; | N, 5.62;<br>N, 5.32. |

HPLC indicated the product contained 88.1% of the S isomer and 6.7% of the R isomer.

Starting materials useful in preparing the compound of this invention, as set forth in the above examples, are synthesized as follows.

Preparation 3

2R,3R 1-Methyl-2-hydroxypropyl 1-isopropyl-9,10-dihydrolysergate

A reaction mixture, prepared from 4 g of 1-isopropyl-9,10-dihydrolysergic acid, 4.05 g of p-toluenesulfonic acid monohydrate and 40 g of 2R,3R-(-)-butandiol (commercially available) was heated to about 60 °C for about 18 hours. At this time, all solids had dissolved and TLC ($CHCl_3$/MeOH/acetic acid 9:3:0.5) showed absence of starting material. The reaction mixture was cooled and was then added to 160 ml of purified water. The pH of the solution was adjusted to about 8.0 with 28% aqueous ammonium hydroxide. The alkaline mixture was stirred and then filtered. The filter cake was rinsed with water. The filter cake, containing 2R,3R 1-methyl-2-hydroxypropyl 1-isopropyl-9,10-dihydrolysergate formed in the above reaction, weighed 3.96 g (80.5% crude yield); MP = 193-200 °C; molecular ion at 384.

| | | | | |
|---|---|---|---|---|
| Analysis: | Calc.:<br>Found: | C, 71.84;<br>C, 71.58; | H, 8.39;<br>H, 8.50; | N, 7.29;<br>N, 7.02. |

The maleate salt was prepared in 92 ml methanol using a slight molar excess of maleic acid. Ether (540 ml) was added to the cooled solution to the point of incipient precipitation. Crystals of the maleate salt which precipitated were collected by filtration. The filter cake was dried; weight = 3.54 g; MP = 182-183.5 °C;

$[\alpha]_d^{25}$ = -58.5 °

| | | | | |
|---|---|---|---|---|
| Analysis: | Calc.:<br>Found: | C, 64.78;<br>C, 64.68; | H, 7.25;<br>H, 7.33; | N, 5.60;<br>N, 5.76. |

An additional 1.03 g of maleate salt were recovered from the filtrate.

Preparation 4

Preparation of 2S,3S 1-Methyl-2-hydroxypropyl 1-isopropyl-9,10-dihydrolysergate

A reaction mixture was prepared from 1.00 g of 1-isopropyl-9,10-dihydrolysergic acid, 1.0 g of p-toluenesulfonic acid, 0.5 g of 2S,3S-O-isopropylidene L-butanediol [prepared by the method of Platner and Rapaport J.A.C.S., 93 1756 (1971)], 0.16 g of water and 15 ml of acetonitrile. The reaction mixture was heated to reflux temperature for about 48 hours. The reaction mixture was cooled and the solids which precipitated were separated by filtration. The filter cake was discarded. The filtrate was evaporated to dryness and the residue partitioned between 50 ml of $(CH_2Cl)_2$ and 50 ml of water. The pH of the aqueous layer was adjusted to about 9.0 with 28% aqueous ammonium hydroxide. The two layers were thoroughly mixed. The layers were separated. The organic layer was emulsified and was therefore washed with 50 ml of brine. The organic layer was dried and the solvent evaporated therefrom. The residue (0.9 g) was purified by HPLC over a C-18 column-(eluate = $CH_3CN/H_2O$ 65:35 plus a trace of $Et_3N$).

2S,3S 1-Methyl-2-hydroxypropyl 1-isopropyl-9,10-dihydrolysergate thus purified was converted to the maleate salt as in the previous preparation. 1.3 g of base plus 0.43 g of maleic acid gave 1.2 g of salt; MP = 194-195.5 °C (with decomposition); molecular ion of base = 384;

$[\alpha]_d^{25}$ = -43.37 °

| | | | | |
|---|---|---|---|---|
| Analysis: | Calc.:<br>Found: | C, 64.78;<br>C, 64.67; | H, 7.25;<br>H, 7.16; | N, 5.60;<br>N, 5.42. |

Example 4

Preparation of Cyclohexyl 1-Isopropyl-9,10-dihydrolysergate (1-Isopropyl-6-methyl-8β-cyclohexyloxycarbonylergoline)

A reaction mixture, prepared from 12.5 g of 1-isopropyl-9,10-dihydrolysergic acid (from Garbrecht, and Lin, United States Patent 3,183,234), 50 ml of cyclohexanol, and 7.6 g of p-toluenesulfonic acid, was heated at about 90°C for 24 hours. The reaction mixture was cooled and the cooled mixture partitioned between $(CH_2Cl)_2$ and dilute aqueous ammonium hydroxide (pH=10). The organic layer was separated and the separated layer washed with water and then dried. Evaporation of the solvent in vacuo yielded a residue comprising cyclohexyl 1-isopropyl-9,10-dihydrolysergate formed in the above reaction. The free base was dissolved in methanol and a molar equivalent of maleic acid added. Ether was added and a crystalline maleate salt was obtained, which salt was separated by filtration. Two recrystallizations from a methanol/ether solvent mixture gave cyclohexyl 1-isopropyl-9,10-dihydrolysergate maleate melting at 203-5°C; $[\alpha]_D^{25}$ = -51.7° (1% methanol); yield = 7.1 g (2 crops); Mass spectrum; m/e at 394 (free base).

| | | | | |
|---|---|---|---|---|
| Analysis: | Calc.:<br>Found: | C, 68.21;<br>C, 67.96; | H, 7.50;<br>H, 7.71; | N, 5.49;<br>N, 5.29. |

Example 5

Preparation of 1-Isopropyl-6-ethyl-8β-cyclohexyloxycarbonylergoline

Following the procedure of Example 10, 1.5 g of 1-isopropylergoline-8β-carboxylic acid were esterified with 15 g of cyclohexanol in the presence of 1.5 g of p-toluenesulfonic acid to yield 1-isopropyl-8β-(cyclohexyloxycarbonyl)ergoline. The product, 1.91 g of an oil, was converted to the maleate salt in a 6:1 ether/ethyl acetate solvent mixture. The salt was isolated by evaporation and then recrystallized from ether/methanol; yield = .93 g of cyclohexyl 1-isopropyl-8β-(cyclohexyloxycarbonyl)ergoline maleate (96.77% pure by HPLC); molecular ion at 380; $[\alpha]^D_{25}$ = -40.64°.

| | | | | |
|---|---|---|---|---|
| Analysis: | Calc.:<br>Found: | C, 67.72;<br>C, 67.95; | H, 7.31;<br>H, 7.54; | N, 5.64;<br>N, 5.36. |

Two grams of 1-isopropyl-8β-cyclohexyloxycarbonylergoline maleate were converted to the free base by partitioning between 100 ml of $(CH_2Cl)_2$ and 100 ml. of saturated aqueous sodium bicarbonate. The free base passed into the organic layer. The organic layer was separated and the solvent evaporated therefrom. The resulting residue was dissolved in 15 ml of DMF, and .67 g of potassium carbonate and .75 g of ethyl iodide added to the solution. This reaction mixture was stirred at ambient temperature for about 3 days at which time HPLC indicated that starting material was no longer present. The reaction mixture was partitioned between 50 ml ethyl acetate and 50 ml of water. The organic layer was separated and the separated layer twice extracted with 50 ml portions of water. The organic layer was dried and the solvent removed by evaporation. The resulting residue (weight = 1.67 g) comprising cyclohexyl 1-isopropyl-6-ethyl-8β-cyclohexyloxycarbonylergolineformed in the above reaction, was converted to the corresponding hydrochloride salt in ethyl acetate solution with gaseous HCl. Two crops of the crystalline salt were recovered by filtration; yield = 1.42 g; purity by HPLC >99.2%. The crystalline fraction was slurried in a THF/ether solvent mixture and the slurry filtered. 1-Isopropyl-6-ethyl-8β-cyclohexyloxycarbonylergoline hydrochloride thus prepared melted above 220°C; yield = 1.33 g; molecular ion at 408.

| | | | | |
|---|---|---|---|---|
| Analysis (block dried); | Calc.:<br>Found: | C, 70.17;<br>C, 70.02; | H, 8.38;<br>H, 8.50; | N, 6.29;<br>N, 6.47. |

Other 6-alkyl derivatives of cyclohexyl 1-isopropyl-8β-cyclohexyloxycarbonylergoline prepared by the above reaction sequence include

1-Isopropyl-6-n-propyl-8β-cyclohexyloxyergoline hydrochloride; yield = 1.14 g (from 2.0 g starting material); mp>220 °C.; purity >98.6%; molecular ion at 422;

| | | | | |
|---|---|---|---|---|
| Analysis (block dried); | Calc.:<br>Found: | C, 70.64;<br>C, 70.41; | H, 8.56;<br>H, 8.51; | N, 6.10;<br>N, 6.36. |

1-Isopropyl-6-n-butyl-8β-cyclohexyloxycarbonyl ergoline hydrochloride; yield = 1.45 g (purity >99.1%); mp >220 °C.; molecular ion at 436;

| | | | | |
|---|---|---|---|---|
| Analysis (block dried); | Calc.:<br>Found: | C, 71.09;<br>C, 70.85; | H, 8.74;<br>H, 8.62; | N, 5.92;<br>N, 5.66. |

Example 6

Preparation of 4-Oxocyclohexyl 1-isopropyl-9,10-dihydrolysergate

Following the procedure of Example 10, 3.12 g of 1-isopropyl-9,10-dihydrolysergic acid were esterified with 1.2 g of 4-oxocyclohexanol in the presence of 4 ml of triethylamine and 3.83 g of methyl 2-chloropyridinium chloride in 10 ml of $CH_2Cl_2$. The reaction mixture was refluxed for 3 hours and stirred overnight at room temperature. Water and $(CH_2Cl)_2$ were added. The organic layer was separated and the solvent evaporated therefrom to leave a brown oil comprising 4-oxocyclohexyl 1-isopropyl-9,10-dihydrolysergate free base formed in the above reaction. The free base was converted to the maleate salt as in Example 11. Recrystallization of the salt from acetone/ether and then methanol/ether gave .05 g of crystalline material salt; .03 g of free base of 95.5% purity were also recovered; molecular ion at 408.

Useful starting material for the above reactions are prepared as follows.

Preparation 5

Preparation of 4-Hydroxycyclohexanone.

Four grams of 4-methoxycyclohex-3-en-1-ol were dissolved in 50 ml of $CHCl_3$. About .01 g of p-toluenesulfonic acid were added and the reaction mixture stirred at room temperature for about 1 hour. The reaction mixture was the washed with 50 ml of water. The $CHCl_3$ layer was separated and dried and the volatile constituents removed therefrom in vacuo. The product of the reaction, 4-oxocyclohexanol, was used without further purification.

Preparation 6

Preparation of 1-Isopropylergoline-8β-carboxylic acid

A reaction mixture was prepared by adding 11.6 ml of 18M sulfuric acid to a mixture of 50 g of 1-isopropyl-9,10-dihydrolysergic acid (prepared according to the procedure of column 3, United States Patent 3,103,234) and 500 ml of methanol. The esterification mixture was stirring overnight at room temperature. TLC (18:6:1 $CHCl_3$/MeOH/acetic acid) indicated the esterification was complete. About 1/3 of the MeOH was removed by evaporation. 600 ml of saturated aqueous sodium bicarbonate were added (pH = 8-9). The alkaline mixture was filtered and the filter cake dried; yield of methyl 1-isopropyl-9,10-dihydrolysergate was 40.1 g (98% pure by LC.).

A reaction mixture was prepared from 39.9 g of methyl 1-isopropyl-9,10-dihydrolysergate, 14.8 g of CNBr and 400 ml of $CH_2Cl_2$. The reaction mixture was stirred at room temperature overnight, by which time

TLC (same solvent system as above) indicated absence of starting material. Evaporation of the volatile constituents gave a solid residue (wt = 46.7 g) comprising methyl 1-isopropyl-6-cyanoergoline-8β-carboxylate formed in the above reaction. The residue was dissolved in 460 ml of refluxing MeOH and the hot solution filtered. Crystals formed in the filtrate, which was then chilled overnight. The crystals were filtered and the filter cake washed with MeOH. Methyl 1-isopropyl-6-cyanoergoline thus prepared was one spot material; yield = 35.3 g. NMR confirmed the structure.

A reaction mixture was prepared by combining 25g of methyl 1-isopropyl-6-cyanoergoline-8β-carboxylate. 8.89 NaOH pellets and 250 ml of ethylene glycol. The reaction mixture was heated in the range 130-40 °C for about 3 hours. 750 ml of water were added. The pH of the resulting solution was adjusted to about 5 with glacial acetic acid (30 mls.). Crystals began to form and the solution was chilled overnight. The crystals were separated by filtration and the filter cake washed with water; yield (after drying) = 19.6 g of 1-isopropylergoline-8β-carboxylic acid, (97.3% pure by HPLC).

This invention also provides novel methods whereby $5HT_2$ receptors are blocked. Such methods are potentially useful in treating disease states in which an excess of circulating serotonin is a major contributing cause. These disease states include hypertension, anorexia nervosa, depression, mania, carcinoid syndrome, migraine and vasospasm. The compounds according to Formula (III) show relatively slight affinity for other receptors, $\alpha_1$, $\alpha_2$, $\beta$, histamine, carbachol etc. and thus are highly selective in their action. Formulations in which a compound of this invention is an active ingredient also form another aspect of this invention.

In order to demonstrate that compounds according to Formula (III) have an extremely high affinity for $5NT_2$ receptors, apparent dissociation constants ($K_B$) as a measure of affinity for $5HT_2$ receptors, expressed as the negative logarithm, have been determined according to the following protocol.

Male Wistar rats (150-300 gram weight) were killed and their external jugular veins and thoracic aortas dissected free of connective tissue, cannulated in situ and placed in a modified Krebs' bicarbonate buffer in a suitable tissue bath. Two L-shaped 30-gauge stainless-steel hypodermic needles were inserted in each cannula and the dissected vessels gently pushed onto the needles. One needle was attached with thread to a stationary glass rod and the other to the transducer. [The procedure employed was that described by Hooker, Calkins and Fleisch, Blood Vessels, 14, 1, (1977) for use with circular smooth muscle preparations.]

The modified Krebs' bicarbonate buffer had the following makeup: (concentrations in millimoles): sodium chloride, 118.2; potassium chloride, 4.6; calcium chloride dihydrate, 1.6; potassium dihydrogenphosphate, 1.2; magnesium sulfate, 1.2; dextrose, 10.0; sodium bicarbonate, 24.8; and water q.s. to 1000 g. The tissue baths were maintained at 37° C. and were aerated with 95% oxygen-5% $CO_2$. An initial optimum resting force of 1 and 4 g was applied to the jugular vein and aorta, respectively. Isometric contractions were recorded as changes in grams of force on a Beckman Dynograph with Statham UC-3 transducers and microscale accessory attachment. Tissues were allowed to equilibrate 1 to 2 hours before exposure to drugs. Control responses to serotonin in the jugular vein and to norepinephrine in the aorta were obtained. The vessels were then incubated with appropriate concentrations of antagonist for one hour. Responses to serotonin or to norepinephrine were then repeated in the presence of the antagonist. Contraction to serotonin was evaluated in the jugular vein since this tissue produces marked responses to serotonin in the absence of alpha receptors -- see Cohen and Wiley, J. Pharm. Exp. Ther., 205, 400 (1978). Alpha receptor antagonist activity was evaluated in the aorta($\alpha_1$) or guinea pig ileum ($\alpha_2$).

Apparent antagonist dissociation constants were determined for each concentration of antagonist according to the following equation:

$$K_B = \frac{[B]}{[\text{dose ratio-1}]}$$

wherein [B] is the concentration of the antagonist and the dose ratio is the $ED_{50}$ of the agonist in the presence of the antagonist divided by the control $ED_{50}$. These results are then expressed as the negative logarithm of $K_B$. The -log $K_B$ values obtained for compounds of this invention are given below in Table 1.

TABLE 1

Apparent Dissociation Constants for $5HT_2$ receptors determined in the rat jugular vein.

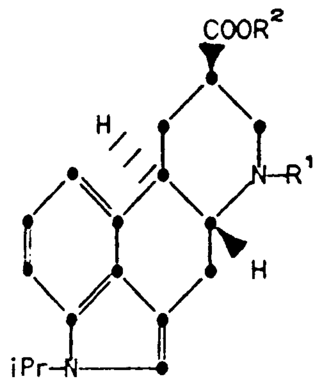

| Compound $R^1$ | $R^2$ | salt | $5HT_2$ -Log $K_b$ ± S.E. |
|---|---|---|---|
| $CH_3$ | cis-4-methoxy cyclohexyl | maleate | 9.54 ± 0.11(13) |
| $CH_3$ | trans-4-methoxy cyclohexyl | maleate | 8.96 ± 0.07(18) |
| $CH_3$ | R-CH($CH_3$)$COCH_3$ | maleate | 9.45 ± .05 |
| $CH_3$ | S-CH($CH_3$)$COCH_3$ | maleate | 9.57 ± .10 |
| $CH_3$ | cyclohexyl | maleate | 8.67 ± S.E. |

Table 1 (Continued)

| Compound $R^1$ | $R^2$ | salt | $5HT_2$ -Log $K_b$ ± S.E. |
|---|---|---|---|
| $CH_3$ | 4-oxocyclohexyl | maleate | 10.01 ± 0.08 |
| $C_2H_5$ | cyclohexyl | HCl | 7.81 ± 0.17 |
| n-$C_3H_7$ | cyclohexyl | HCl | 7.07 ± 0.05 |
| n-$C_4H_9$ | cyclohexyl | HCl | 7.4 ± 0.19 |

In mammals, hypertension may be mediated through $5HT_2$ receptors. Thus, compounds of Formula (III) would be expected to lower blood pressure in humans as does ketanserin, another $5HT_2$ blocker, but without the side effects attributable to alpha adrenergic receptor blockade of ketanserin.

In carrying out our novel therapeutic process, a pharmaceutically-acceptable salt of a drug according to Formula (III) above formed with a non-toxic acid is administered orally or parenterally to a mammal with an excess of circulatory serotonin in which mammal is desirable to block $5HT_2$ receptors in order to alleviate symptoms attributable to excessive serotonin levels such as high blood pressure and migraine. For parenteral administration, a water soluble salt of the drug is dissolved in an isotonic salt solution and administered by the i.v. route. For oral administration, a pharmaceutically-acceptable salt of the drug is mixed with standard pharmaceutical excipients such as starch and loaded into capsules or made into tablets, each containing 0.1 to 100 mg of active drug. Dosage levels of from 0.1-10 mg/kg have been found to be effective in blocking $5HT_2$ receptors. Thus, the oral dosage would be administered 2-4 times per day, giving a daily dosage range of about .003 to about 10.0 mg./kg. per day.

Other oral dosage forms, suspensions, elixirs and tablets, can also be utilized and are preparable by standard procedures.

## Claims

**Claims for the following Contracting States : BE, CH, DE, FR, GB, IT, LI, LU, NL, SE**

**1.** A compound of Formula (III):

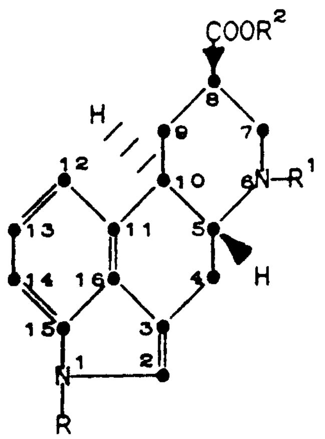

(III)

wherein R is primary or secondary $C_1$-$C_8$ alkyl, $C_2$ -$C_4$ alkenyl-$CH_2$, $C_3$-$C_8$ cycloalkyl or $C_3$-$C_6$ cycloalkyl-substituted $C_1$-$C_5$ primary or secondary alkyl, the total number of carbon atoms in R not to exceed 8; $R^1$ is allyl, H, or $C_1$-$C_4$ straight chain alkyl, and $R^2$ is $C_1$-$C_3$ alkoxy-$C_5$-$C_7$ cycloalkyl; $C_3$-$C_7$ ketoalkyl; or $C_5$-$C_7$ cycloalkyl or keto-substituted $C_5$-$C_7$ cycloalkyl; or a pharmaceutically-acceptable salt thereof.

**2.** A compound as claimed in claim 1, wherein $R^2$ is $C_1$-$C_3$ alkoxy-$C_5$-$C_7$ cycloalkyl.

**3.** A compound as claimed in claim 1, wherein $R^2$ is $C_3$-$C_7$ ketoalkyl.

**4.** A compound as claimed in claim 1, wherein $R^2$ is $C_5$-$C_7$ cycloalkyl or keto substituted $C_5$-$C_7$cycloalkyl.

**5.** A compound as claimed in any one of claims 1 to 4, wherein R is isopropyl.

**6.** A compound as claimed in any one of claims 1 to 5, wherein $R^1$ is methyl.

**7.** A compound as claimed in any one of claims 1, 2, 5, and 6, wherein $R^2$ is trans 4-methoxycyclohexyl.

**8.** Trans-4-methoxycyclohexyl 1-isopropyl-9,10-dihydrolysergate, or a pharmaceutically acceptable salt thereof.

**9.** Trans-4-methoxycyclohexyl 1-isopropyl-9,10-dihydrolysergate maleate.

**10.** A process for preparing a compound of Formula (III) as defined in any one of claims 1 to 9, which comprises:

A) esterifying the 8-carboxylic acid function of a compound of Formula (V):

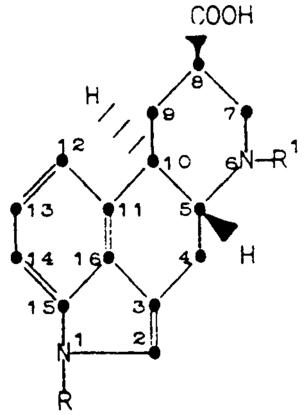

(V)

wherein R and $R^1$, are as defined in claim 1; or

B) oxidizing a compound of Formula (VI):

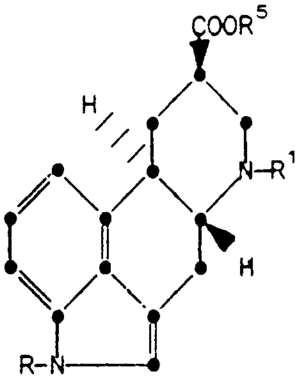

(VI)

wherein $R^5$ is a $C_3$-$C_6$ hydroxyalkyl group;

C) alkylating a compound of Formula (III):

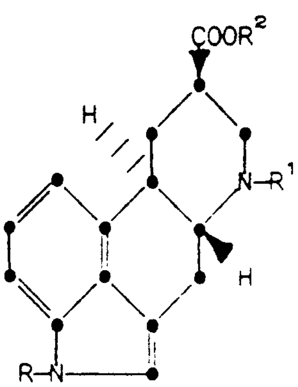

(III)

wherein only one of R and $R^1$ is hydrogen, or,

D) hydrogenating a compound of Formula (VIII):

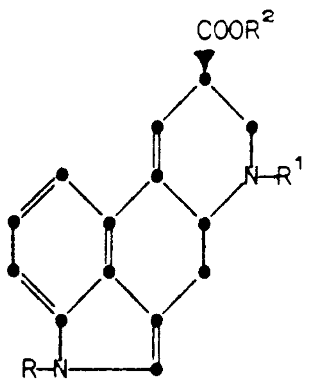

(VIII)

**11.** A process according to claim 10 for preparing a compound of Formula (III), which comprises: reacting a compound of Formula (V) with a compound of the formula $R^2$-O-$SO_2$-Z, wherein Z is $C_1$-$C_3$ alkyl, phenyl or phenyl substituted by $C_1$-$C_3$ alkyl, nitro, halo, or $C_1$-$C_3$ alkoxy, in the presence of a base, wherein $R^2$ is as defined in claim 1.

**12.** A compound of Formula (III), or a pharmaceutically-acceptable salt thereof, as claimed in any one of claims 1 to 9, for use as a $5HT_2$ antagonist.

**13.** Trans-4-methoxycyclohexyl 1-isopropyl-9,10-dihydrolysergate maleate for use in treating migraine.

**14.** A pharmaceutical formulation comprising as an active ingredient a compound of Formula (III), or a pharmaceutically-acceptable acid-addition salt thereof, as claimed in any one of claims 1 to 9, associated with one or more pharmaceutically-acceptable carriers therefor.

**Claims for the following Contracting State : AT**

**1.** A process for preparing a compound of Formula (III):

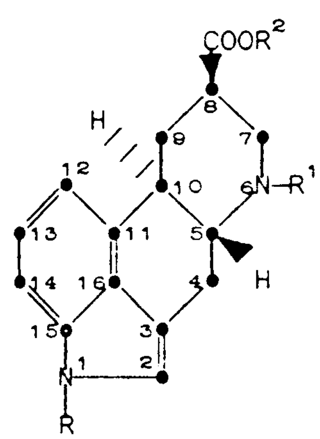

(III)

wherein R is primary or secondary $C_1$-$C_8$ alkyl, $C_2$-$C_4$ alkenyl-$CH_2$, $C_3$-$C_8$ cycloalkyl or $C_3$-$C_6$ cycloalkyl-substituted $C_1$-$C_5$ primary or secondary alkyl, the total number of carbon atoms in R not to exceed 8; $R^1$ is allyl, H, or $C_1$-$C_4$ straight chain alkyl, and $R^2$ is $C_1$-$C_3$ alkoxy-$C_5$-$C_7$ cycloalkyl; $C_3$-$C_7$ ketoalkyl; or $C_5$-$C_7$ cycloalkyl or keto-substituted $C_5$-$C_7$ cycloalkyl; or a pharmaceutically-acceptable salt thereof,
which comprises:

A) esterifying the 8-carboxylic acid function of a compound of Formula (V):

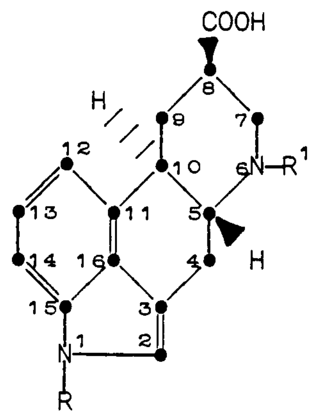

(V)

wherein R and $R^1$, are as defined in claim 1; or

B) oxidizing a compound of Formula (VI):

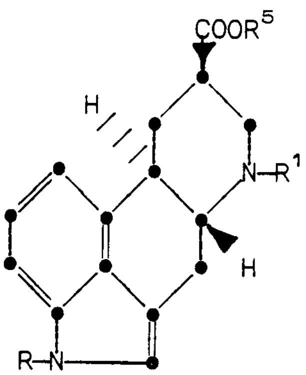

(VI)

wherein $R^5$ is a $C_3$-$C_6$ hydroxyalkyl group

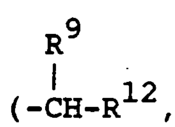

wherein $R^9$ is H, methyl, or ethyl, and $R^{12}$ is a $C_2$-$C_5$ alkyl group containing a secondary hydroxy group).

C) alkylating a compound of Formula (III):

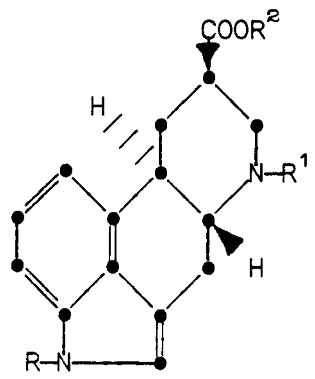

(III)

wherein only one of R and $R^1$ is hydrogen, or,
D) hydrogenating a compound of Formula (VIII):

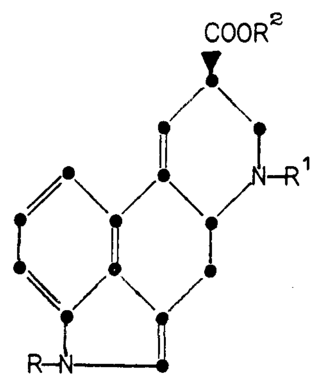

(VIII)

2. A process according to claim 1 for preparing a compound of Formula (III), which comprises: reacting a compound of Formula (V) with a compound of the formula $R^2$-O-$SO_2$-Z, wherein Z is $C_1$-$C_3$ alkyl, phenyl or phenyl substituted by $C_1$-$C_3$ alkyl, nitro, halo, or $C_1$-$C_3$ alkoxy, in the presence of a base, wherein $R^2$ is as defined in claim 1.

3. A process according to claim 1 or 2, wherein $R^2$ is $C_1$-$C_3$ alkoxy-$C_5$-$C_7$ cycloalkyl.

4. A process according to claim 1 or 2, wherein $R^2$ is $C_3$-$C_7$ ketoalkyl.

5. A process according to claim 1 or 2, wherein $R^2$ is $C_5$-$C_7$ cycloalkyl or keto substituted $C_5$-$C_7$ cycloalkyl.

6. A process according to any one of claims 1 to 4, wherein R is isopropyl.

7. A process according to any one of claims 1 to 5, wherein $R^1$ is methyl.

8. A process according to any one of claims 1, 2, 5, and 6, wherein $R^2$ is trans 4-methoxycyclohexyl.

9. A process according to claim 1 or 2, for preparing trans-4-methoxycyclohexyl 1-isopropyl-9,10-dihydrolysergate, or a pharmaceutically acceptable salt thereof.

**Patentansprüche**

**Patentansprüche für folgende Vertragsstaaten : BE, CH, DE, FR, GB, IT, LI, LU, NL, SE**

**1.** Verbindung der Formel (III):

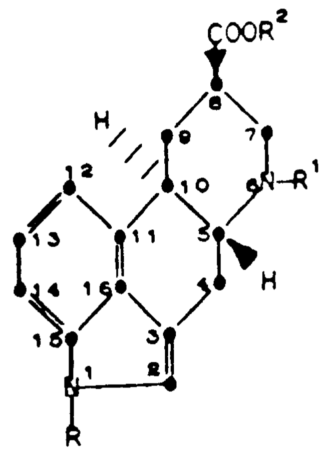

(III)

worin R ein primärer oder sekundärer $C_1$-$C_8$-Alkyl-, $C_2$-$C_4$-Alkenyl-$CH_2$-, $C_3$-$C_8$-Cycloalkyl- oder $c_3$-$C_6$-cycloalkylsubstituierter primärer oder sekundärer $C_1$-$C_5$-Alkylrest ist, wobei die Gesamtzahl von Kohlenstoffatomen in dem Rest R nicht höher als 8 ist; $R^1$ ein Allylrest, H oder ein geradkettiger $C_1$-$C_4$-Alkylrest ist; und $R^2$ ein $C_1$-$C_3$-Alkoxy-$C_5$-$C_7$-cycloalkylrest; $C_3$-$C_7$-Ketoalkylrest oder $C_5$-$C_7$-Cycloalkylrest oder ein ketosubstituierter $C_5$-$C_7$-Cycloalkylrest ist; und pharmazeutisch annehmbare Salze davon.

**2.** Verbindung nach Anspruch 1, worin $R^2$ ein $C_1$-$C_3$-Alkoxy-$C_5$-$C_7$-cycloalkylrest ist.

**3.** Verbindung nach Anspruch 1, worin $R^2$ ein $C_3$-$C_7$-Ketoalkylrest ist.

**4.** Verbindung nach Anspruch 1, worin $R^2$ ein $C_5$-$C_7$-Cycloalkylrest oder ein mit Keto substituierter $C_5$-$C_7$-Cycloalkylrest ist.

**5.** Verbindung nach einem der Ansprüche 1 bis 4, worin R ein Isopropylrest ist.

**6.** Verbindung nach einem der Ansprüche 1 bis 5, worin $R^1$ ein Methylrest ist.

**7.** Verbindung nach einem der Ansprüche 1, 2, 5 und 6, worin $R^2$ ein trans-4-Methoxycyclohexylrest ist.

**8.** trans-4-Methoxycyclohexyl-1-isopropyl-9,10-dihydrolysergat oder ein pharmazeutisch annehmbares Salz davon.

**9.** trans-4-Methoxycyclohexyl-1-isopropyl-9,10-dihydrolysergatmaleat.

**10.** Verfahren zur Herstellung einer Verbindung der Formel (III), wie in einem der Ansprüche 1 bis 9 definiert, umfassend, daß man:

(A) die Carbonsäurefunktion an Position 8 einer Verbindung der Formel (V):

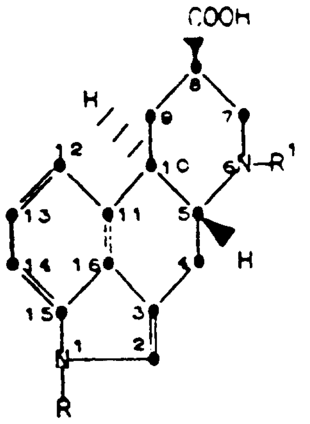

(V)

worin R und $R^1$ wie in Anspruch 1 definiert sind, verestert; oder
(B) eine Verbindung der Formel (VI):

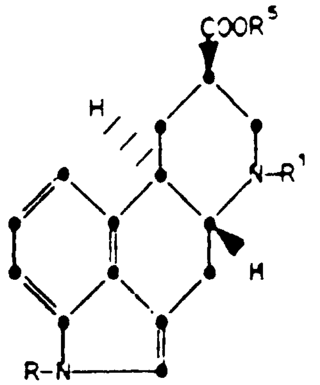

(VI)

worin $R^5$ eine $C_3$-$C_6$-Hydroxyalkylgruppe ist, oxidiert;
(C) eine Verbindung der Formel (III):

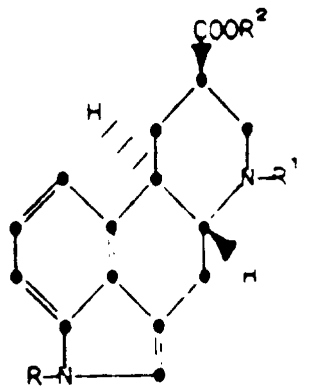

(III)

worin nur einer der Reste R und $R^1$ Wasserstoff ist, alkyliert; oder

(D) eine Verbindung der Formel (VIII):

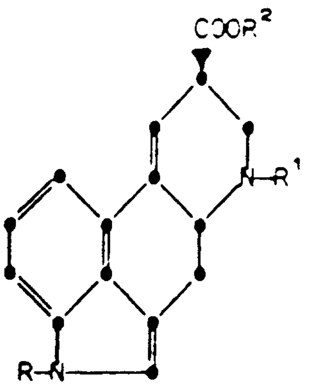

(VIII)

hydriert.

**11.** Verfahren nach Anspruch 10 zur Herstellung einer Verbindung der Formel (III), umfassend, daß man eine Verbindung der Formel (V) mit einer Verbindung der Formel $R^2$-O-$SO_2$-Z, worin Z ein $C_1$-$C_3$-Alkyl-, Phenyl- oder mit $C_1$-$C_3$-Alkyl-, Nitro-, Halogen- oder $C_1$-$C_3$-Alkoxyresten substituierter Phenylrest ist, in Gegenwart einer Base umsetzt, wobei $R^2$ wie in Anspruch 1 definiert ist.

**12.** Verbindung der Formel (III) oder ein pharmazeutisch annehmbares Salz davon, wie in einem der Ansprüche 1 bis 9 beansprucht, zur Verwendung als $5HT_2$-Antagonist.

**13.** trans-4-Methoxycyclohexyl-1-isopropyl-9,10-dihydrolysergatmaleat zur Verwendung zur Behandlung von Migräne.

**14.** Pharmazeutische Formulierung umfassend als aktiven Inhaltsstoff eine Verbindung der Formel (III) oder ein pharmazeutisch annehmbares Säureadditionssalz davon nach einem der Ansprüche 1 bis 9 zusammen mit einem oder mehreren pharmazeutisch annehmbaren Trägern.

**Patentansprüche für folgenden Vertragsstaat : AT**

**1.** Verfahren zur Herstellung einer Verbindung der Formel (III):

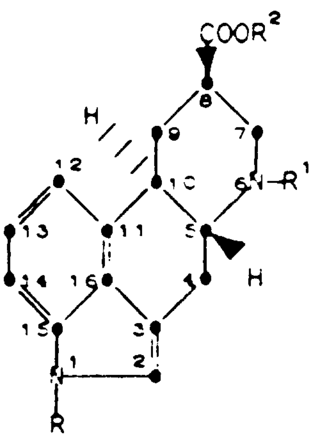

(III)

worin R ein primärer oder sekundärer $C_1$-$C_8$-Alkyl-, $C_2$-$C_4$-Alkenyl-$CH_2$-, $C_3$-$C_8$-Cycloalkyl- oder $C_3$-$C_6$-cycloalkylsubstituierter primärer oder sekundärer $C_1$-$C_5$-Alkylrest ist, wobei die Gesamtzahl von Kohlenstoffatomen in dem Rest R nicht höher als 8 ist; $R^1$ ein Allylrest, H oder ein geradkettiger $C_1$-$C_4$-

Alkylrest ist; und $R^2$ ein $C_1$-$C_3$-Alkoxy-$C_5$-$C_7$-cycloalkylrest; $C_3$-$C_7$-Ketoalkylrest oder $C_5$-$C_7$-Cycloalkylrest oder ketosubstituierter $C_5$-$C_7$-Cycloalkylrest ist; oder pharamazeutisch annehmbarer Salze davon, umfassend, daß man

(A) die Carbonsäurefunktion an Position 8 einer Verbindung der Formel (V):

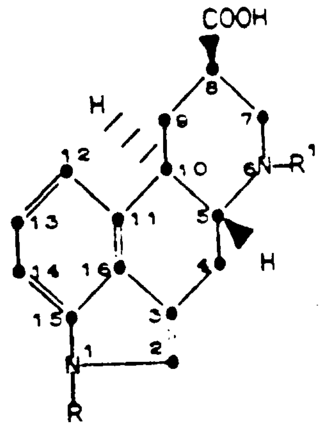

(V)

worin R und $R^1$ wie in Anspruch 1 definiert sind, verestert; oder

(B) eine Verbindung der Formel (VI):

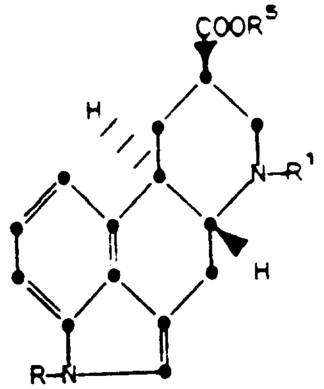

(VI)

worin $R^5$ eine $C_3$-$C_6$-Hydroxyalkylgruppe

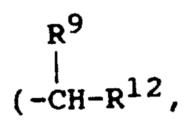

worin $R^9$ H, Methyl oder Ethyl ist und $R^{12}$ eine $C_2$-$C_5$-Alkylgruppe ist, die eine sekundäre Hydroxygruppe enthält) ist, oxidiert;

(C) eine Verbindung der Formel (III):

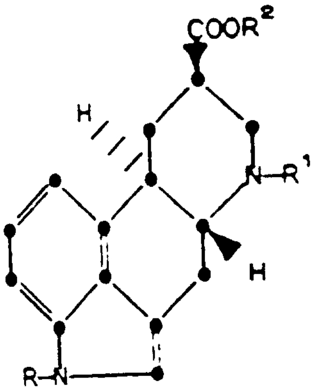

(III)

worin nur einer der Reste R und $R^1$ Wasserstoff ist, alkyliert; oder
(D) eine Verbindung der Formel (VIII):

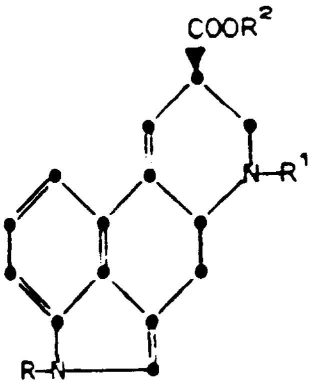

(VIII)

hydriert.

**2.** Verfahren nach Anspruch 1 zur Herstellung einer Verbindung der Formel (III), umfassend, daß man eine Verbindung der Formel (V) mit einer Verbindung der Formel $R^2$-O-$SO_2$-Z, worin Z ein $C_1$-$C_3$-Alkyl-, Phenyl- oder mit $C_1$-$C_3$-Alkyl-, Nitro-, Halogen- oder $C_1$-$C_3$-Alkoxyresten substituierter Phenylrest ist, in Gegenwart einer Base umsetzt, wobei $R^2$ wie in Anspruch 1 definiert ist.

**3.** Verfahren nach Anspruch 1 oder 2, worin $R^2$ ein $C_1$-$C_3$-Alkoxy-$C_5$-$C_7$-cycloalkylrest ist.

**4.** Verfahren nach Anspruch 1 oder 2, worin $R^2$ ein $C_3$-$C_7$-Ketoalkylrest ist.

**5.** Verfahren nach Anspruch 1 oder 2, worin $R^2$ ein $C_5$-$C_7$-Cycloalkylrest oder ein mit Keto substituierter $C_5$-$C_7$-Cycloalkylrest ist.

**6.** Verfahren nach einem der Ansprüche 1 bis 4, worin R ein Isopropylrest ist.

**7.** Verfahren nach einem der Ansprüche 1 bis 5, worin $R^1$ ein Methylrest ist.

**8.** Verfahren nach einem der Ansprüche 1, 2, 5 und 6, worin $R^2$ ein trans-4-Methoxycyclohexylrest ist.

**9.** Verfahren nach Anspruch 1 oder 2 zur Herstellung von trans4-Methoxycyclohexyl-1-isopropyl-9,10-dihydrolysergat oder einem pharmazeutisch annehmbaren Salz davon.

**Revendications**

**Revendications pour les Etats contractants suivants : BE, CH, DE, FR, GB, IT, LI, LU, NL, SE**

**1.** Composé de formule (III)

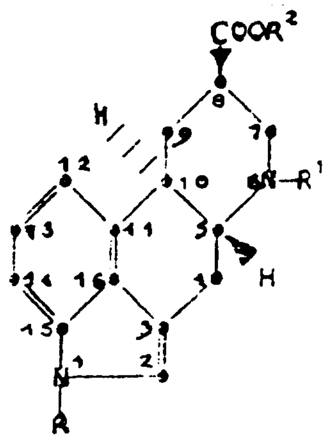

(III)

dans laquelle R représente un groupe alkyle primaire ou secondaire en $C_1$-$C_8$, un groupe alcényl(en $C_2$-$C_4$)-$CH_2$, un groupe cycloalkyle en $C_3$-$C_8$ ou un groupe alkyle primaire ou secondaire en $C_1$-$C_5$ substitué par un groupe cycloalkyle en $C_3$-$C_6$, le nombre total des atomes de carbone dans R ne devant pas dépasser 8; $R^1$ représente un groupe allyle, H ou un groupe alkyle en $C_1$-$C_4$ à chaîne droite; et $R^2$ représente un groupe alcoxy(en $C_1$-$C_3$)cycloalkyle en $C_5$-$C_7$; un groupe cétoalkyle en $C_3$-$C_7$; ou encore un groupe cycloalkyle en $C_5$-$C_7$ ou un groupe cycloalkyle en $C_5$-$C_7$ substitué par un groupe céto; ou bien un de ses sels pharmaceutiquement acceptables.

**2.** Composé selon la revendication 1, dans lequel $R^2$ représente un groupe alcoxy(en $C_1$-$C_3$)cycloalkyle en $C_5$-$C_7$.

**3.** Composé selon la revendication 1, dans lequel $R^2$ représente un groupe cétoalkyle en $C_3$-$C_7$.

**4.** Composé selon la revendication 1, dans lequel $R^2$ représente un groupe cycloalkyle en $C_5$-$C_7$ ou un groupe cycloalkyle en $C_5$-$C_7$ substitué par un groupe céto.

**5.** Composé selon l'une quelconque des revendications 1 à 4, dans lequel R représente un groupe isopropyle.

**6.** Composé selon l'une quelconque des revendications 1 à 5, dans lequel $R^1$ représente un groupe méthyle.

**7.** Composé selon l'une quelconque des revendications 1, 2, 5 et 6, dans lequel $R^2$ représente un groupe trans-4-méthoxycyclohexyle.

**8.** 1-isopropyl-9,10-dihydrolysergate de trans-4-méthoxycyclohexyle ou un de ses sels pharmaceutiquement acceptables.

**9.** 1-isopropyl-9,10-dihydrolysergate maléate de trans-4-méthoxycyclohexyle.

**10.** Procédé pour préparer un composé de formule (III) tel que défini dans l'une quelconque des revendications 1 à 9, qui comprend le fait de :

A) estérifier la fonction acide 8-carboxylique d'un composé de formule (V)

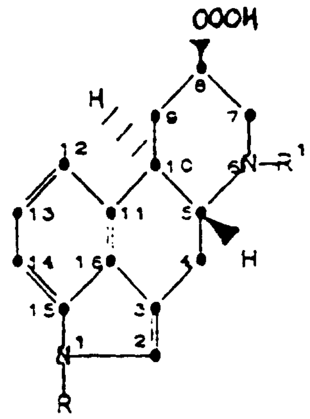

(V)

dans laquelle R et $R^1$ sont tels que définis à la revendication 1; ou
B) oxyder un composé de formule (VI)

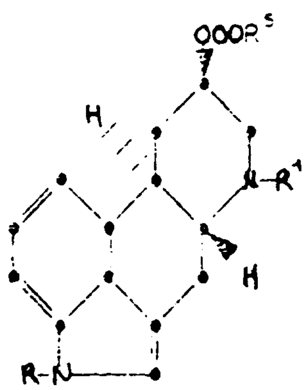

(VI)

dans laquelle $R^5$ représente un groupe hydroxyalkyle en $C_3$-$C_6$;
C) alkyler un composer de formule (III)

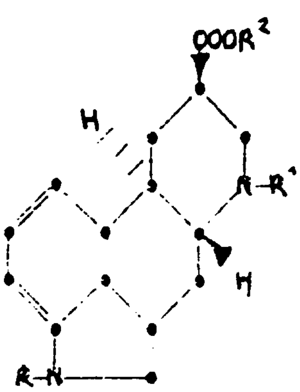

(III)

dans laquelle seulement un des radicaux R et $R^1$ représente un atome d'hydrogène; ou

D) hydrogéner un composé de formule (VIII)

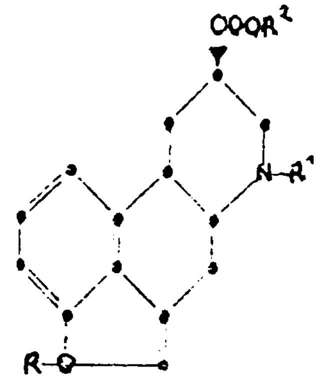

(VIII)

**11.** Procédé selon la revendication 10, pour préparer un composé de formule (III), qui comprend le fait de : faire réagir un composé de formule (V) avec un composé de formule $R^2$-O-$SO_2$-Z où Z représente un groupe alkyle en $C_1$-$C_3$, un groupe phényle ou un groupe phényle substitué par un groupe alkyle en $C_1$-$C_3$, par un groupe nitro, par un atome d'halogène ou par un groupe alcoxy en $C_1$-$C_3$, en présence d'une base, dans laquelle $R^2$ est tel que défini à la revendication 1.

**12.** Composé de formule (III) ou un de ses sels pharmaceutiquement acceptables, tel que revendiqué dans l'une quelconque des revendications 1 à 9, pour être utilisé comme antagoniste des $5HT_2$.

**13.** 1-isopropyl-9,10-dihydrolysergate maléate de trans-4-méthoxycyclohexyle pour être utilisé dans le traitement de la migraine.

**14.** Formulation pharmaceutique comprenant, comme ingrédient actif, un composé de formule (III) ou un de ses sels d'addition d'acides pharmaceutiquement acceptables, tel que revendiqué dans l'une quelconque des revendications 1 à 9, en association avec un ou plusieurs supports pharmaceutiquement acceptables pour l'ingrédient.

**Revendications pour l'Etat contractant suivant : AT**

**1.** Procédé pour préparer un composé de formule (III)

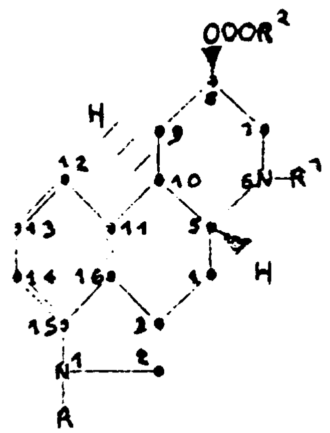

(III)

dans laquelle R représente un groupe alkyle primaire ou secondaire en $C_1$-$C_8$, un groupe alcényl(en $C_2$-$C_4$)-$CH_2$, un groupe cycloalkyle en $C_3$-$C_8$ ou un groupe alkyle primaire ou secondaire en $C_1$-$C_5$ substitué par un groupe cycloalkyle en $C_3$-$C_6$, le nombre total des atomes de carbone dans R ne devant pas dépasser 8; $R^1$ représente un groupe allyle, H ou un groupe alkyle en $C_1$-$C_4$ à chaîne

droite; et $R^2$ représente un groupe alcoxy(en $C_1$-$C_3$)cycloalkyle en $C_5$-$C_7$; un groupe cétoalkyle en $C_3$-$C_7$; ou encore un groupe cycloalkyle en $C_5$-$C_7$ ou un groupe cycloalkyle en $C_5$-$C_7$ substitué par un groupe céto; ou bien un de ses sels pharmaceutiquement acceptables, qui comprend le fait de

A) estérifier la fonction acide 8-carboxylique d'un composé de formule (V)

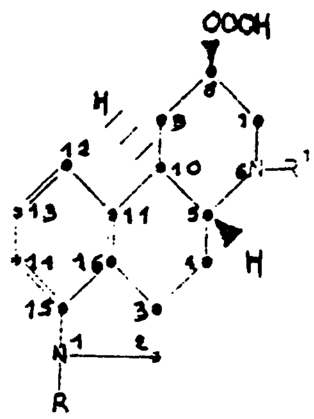

(V)

dans laquelle R et $R^1$ sont tels que définis à la revendication 1; ou

B) oxyder un composé de formule (VI)

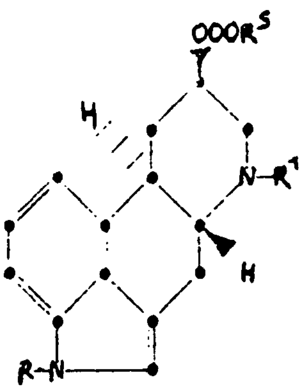

(VI)

dans laquelle $R^5$ représente un groupe hydroxyalkyle en $C_3$-$C_6$

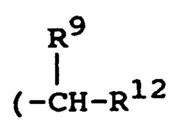

où $R^9$ représente H, un groupe méthyle ou un groupe éthyle, et $R^{12}$ représente un groupe alkyle en $C_2$-$C_5$ contenant un groupe hydroxyle secondaire);

C) alkyler un composer de formule (III)

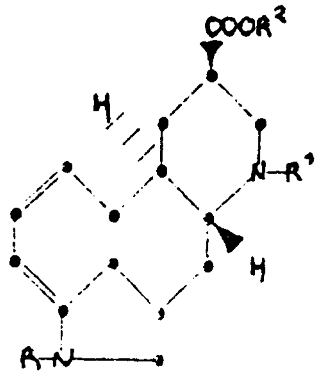

(III)

dans laquelle seulement un des radicaux R et $R^1$ représente un atome d'hydrogène; ou

D) hydrogéner un composé de formule (VIII)

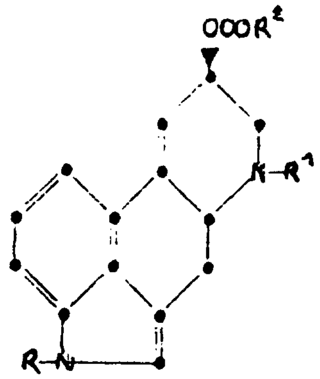

(VIII)

2. Procédé selon la revendication 1, pour préparer un composé de formule (III), qui comprend le fait de : faire réagir un composé de formule (V) avec un composé de formule $R^2$-O-$SO_2$-Z où Z représente un groupe alkyle en $C_1$-$C_3$, un groupe phényle ou un groupe phényle substitué par un groupe alkyle en $C_1$-$C_3$, par un groupe nitro, par un atome d'halogène ou par un groupe alcoxy en $C_1$-$C_3$, en présence d'une base, dans laquelle $R^2$ est tel que défini à la revendication 1.

3. Procédé selon la revendication 1 ou 2, dans lequel $R^2$ représente un groupe alcoxy(en $C_1$-$C_3$)-cycloalkyle en $C_5$-$C_7$.

4. Procédé selon la revendication 1 ou 2, dans lequel $R^2$ représente un groupe cétoalkyle en $C_3$-$C_7$.

5. Procédé selon la revendication 1 ou 2, dans lequel $R^2$ représente un groupe cycloalkyle en $C_5$-$C_7$ ou un groupe cycloalkyle en $C_5$-$C_7$ substitué par un groupe céto.

6. Procédé selon l'une quelconque des revendications 1 à 4, dans lequel R représente un groupe isopropyle.

7. Procédé selon l'une quelconque des revendications 1 à 5, dans lequel $R^1$ représente un groupe méthyle.

8. Procédé selon l'une quelconque des revendications 1, 2, 5 et 6, dans lequel $R^2$ représente un groupe trans-4-méthoxycyclohexyle.

9. Procédé selon la revendication 1 ou 2, pour préparer le 1-isopropyl-9,10-dihydrolysergate de trans-4-méthoxycyclohexyle ou un de ses sels pharmaceutiquement acceptables.